(19) Europäisches Patentamt
European Patent Office
Office européen des brevets

(11) Veröffentlichungsnummer: **0 050 872**
**A1**

(12) # EUROPÄISCHE PATENTANMELDUNG

(21) Anmeldenummer: 81108925.9

(22) Anmeldetag: 26.10.81

(51) Int. Cl.³: C 07 C 49/83
C 09 B 3/04

(30) Priorität: 29.10.80 CH 8048/80

(43) Veröffentlichungstag der Anmeldung: 05.05.82 Patentblatt 82/18

(84) Benannte Vertragsstaaten: CH DE FR GB IT LI

(71) Anmelder: CIBA-GEIGY AG
Patentabteilung Postfach
CH-4002 Basel(CH)

(72) Erfinder: Scherrer, Walter
Lehenmattstrasse 189
CH-4052 Basel(CH)

(72) Erfinder: Portmann, Robert, Dr.
Unterer Rütschetenweg 38
CH-4133 Pratteln(CH)

(74) Vertreter: Zumstein sen., Fritz, Dr. et al,
Bräuhausstrasse 4
D-8000 München 2(DE)

(54) **Verfahren zur Herstellung von 2-Methoxybenzanthronen.**

(57) Die Erfindung betrifft ein neues Verfahren zur Herstellung von 2-Methoxybenzanthronen ausgehend von 1-Aminoanthrachinonen, das sich dadurch auszeichnet, dass durch Anwendung eines speziellen Reaktionsmediums, nämlich eines Dimethylalkanphosphonats, das zugleich Methylierungsmittel in einer der Reaktionsstufen ist, die Reaktion von Anfang bis zum Ende nach dem Prinzip der Eintopfreaktion d.h. ohne Isolierung von Zwischenstufen durchgeführt wird.

Die nach diesem Verfahren erhaltenen 2-Methoxybenzanthrone sind wichtige Küpenfarbstoffzwischenprodukte.

EP 0 050 872 A1

0050872



CIBA-GEIGY AG
Basel (Schweiz)

1-13123/-

Verfahren zur Herstellung von 2-Methoxybenzanthronen

Die vorliegende Erfindung betrifft ein neues Verfahren zur Herstellung von 2-Methoxybenzanthronen in einer Eintopfreaktion, wonach man ausgehend von 1-Aminoanthrachinon oder dessen Derivaten, über das Anthrachinon-1-diazoniumsalz; dessen Umsetzungsprodukt mit einem Propenderivat nach Meerwein; Cyclisierung und schliesslich Methylierung dieses Produktes das entsprechende 2-Methoxybenzanthron erhält. Die Reaktionsfolge wird beginnend mit dem 1-Aminoanthrachinon oder dessen Diazotierungsprodukt, ohne Isolieren von Zwischenstufen, in einem Dimethylalkanphosphonat, welches Reaktionsmedium und Methylierungsreagenz zugleich ist, durchgeführt.

Die nach dem erfindungsgemässen Verfahren herstellbaren 2-Methoxybenzanthrone sind wertvolle Zwischenprodukte für die Fabrikation von Farbstoffen und Pigmenten.

2-Methoxybenzanthron ist z.B. ein wichtiges Küpenfarbstoff-Zwischenprodukt. So liefert die 5-stündige Alkalischmelze von 2-Methoxybenzanthron mit Kaliumhydroxid und Natriummethylat in Polyäthylenglykol 400 als Lösungsmittel bei 130-135°C unter Stickstoff und anschliessende Oxidation mit Luftsauerstoff und übliche Aufarbeitung in hoher Reinheit den Küpenfarbstoff der Formel

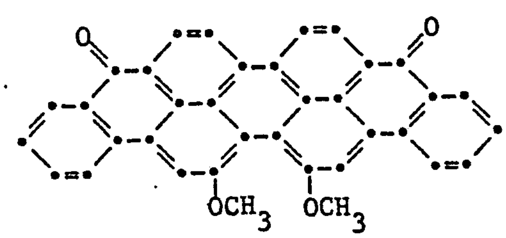

Das neue Eintopfverfahren ist gegenüber dem bekannten Verfahren, das z.B. im J. Chem. Soc. 1948 S. 1088-9 beschrieben wird, recht einfach durchführbar und fortschrittlich.

Das bekannte, von Benzanthron ausgehende Verfahren, umfasst eine Vielzahl von Reaktionsstufen, die zudem jeweils meist isoliert werden müssen, und zwar: die Nitrierung, Reduktion, Acetylierung, wobei 3-Acetamidobenzanthron erhalten wird, das zunächst in der unerwünschten 3-Stellung substituiert ist und aus dem durch Nitrierung und Hydrolysierung 2-Nitro-3-aminobenzanthron hergestellt wird, welches durch Diazotieren und Reduzieren in das gewünschte 2-Hydroxybenzanthron überführt wird. Dieses wird dann zum 2-Methoxybenzanthron methyliert. Es ist verständlich, dass bei der grossen Anzahl von 8 Reaktionsstufen, abgesehen vom erheblichen Anfall an Neben- bzw. Abfallprodukten, die Reaktionsausbeute entsprechend klein ausfällt.

Die der vorliegenden Erfindung zugrundeliegende Aufgabe bestand darin, ein neues Verfahren zur Herstellung von 2-Methoxybenzanthron und dessen Substitutionsprodukten zu finden, welches die zuvor genannten Nachteile nicht aufweist. Es wurde nun gefunden, dass man bei Verwendung von Dimethylalkanphosphonaten als Reaktionsmedium, die zugleich auch Methylierungsmittel sind, überraschenderweise 2-Methoxybenzanthron ohne Isolierung der Zwischenprodukte auf sehr einfache Art und Weise in hoher Ausbeute und Reinheit herstellen kann.

Gegenstand der Erfindung ist somit ein Verfahren zur Herstellung von 2-Methoxybenzanthronen ausgehend von 1-Aminoanthrachinon der Formel I

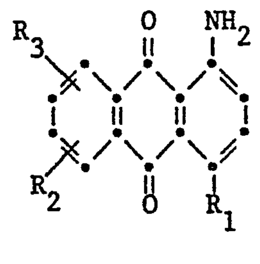

(I)

worin $R_1$, $R_2$ und $R_3$ unabhängig voneinander Wasserstoff- oder Chloratome, Hydroxyl-, Methoxy- oder die Benzamidogruppe bedeuten, in einem Dimethylalkanphosphonat als Reaktionsmedium, das zugleich auch Methylierungsreagenz ist.

Das gegebenenfalls substituierte 1-Aminoanthrachinon der Formel I wird zunächst diazotiert und das Diazoniumsalz mit einem Propenderivat der Formel 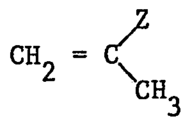, worin Z ein elektronanziehender Substituent ist, in einer Meerwein-Reaktion umgesetzt. Das Reaktionsprodukt wird durch Einwirken einer Base zum 2-Hydroxybenzanthron cyclisiert und die Hydroxygruppe im abschliessenden Reaktionsschritt methyliert.

Das neue Verfahren zeichnet sich dadurch aus, dass die Reaktionsfolge ausgehend vom 1-Aminoanthrachinon der Formel I oder dessen Diazotierungsprodukt, ohne Isolieren von Zwischenstufen in einer Eintopfreaktion durchgeführt wird.

Das Verfahren soll anhand des folgenden Formelschemas veranschaulicht werden, wobei als Ausgangsmaterial 1-Aminoanthrachinon und als Propenderivat Methacrylnitril eingesetzt wird.

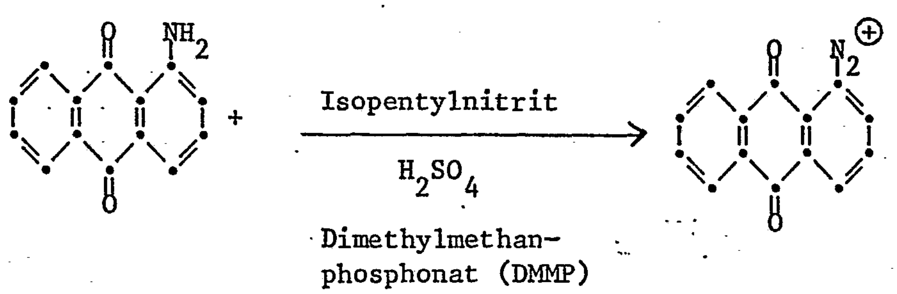

(1)

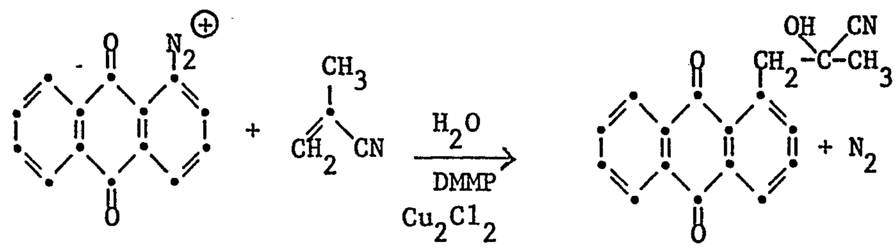

(2)

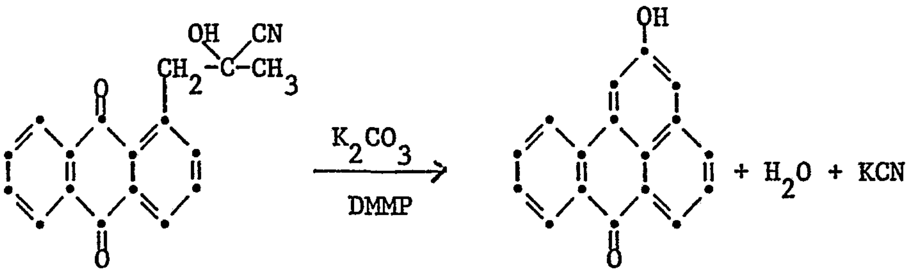

(3)

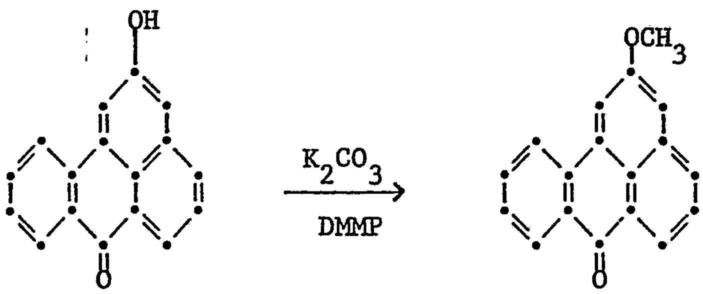

(4)

Als Reaktionsmedium werden Dimethylalkanphosphonate angewandt, bei denen der Alkanrest 1 bis 4 C-Atome aufweist, vor allem solche mit 1-2 C-Atomen und insbesondere das Dimethylmethanphosphonat der Formel

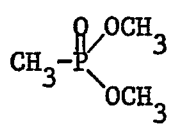
, kurz DMMP genannt.

Als Diazotierungsagentien werden Salpetrigsäureester, insbesondere solche von primären oder sekundären Alkoholen mit 1 bis 10 Kohlenstoffatomen verwendet. Beispiele solcher Ester sind die flüssigen Nitrite von Isopropyl-, Butyl-, Pentyl-, Isopentyl-, Heptyl- und Decylalkohol sowie von Benzylalkohol, mit einem Siedepunkt über 50°C; vorteilhaft verwendet man die Ester von Alkoholen mit 1 bis 5 Kohlenstoffatomen wie z.B. Methyl-, Aethyl-, Propyl-, Isopropyl-, Isobutyl- oder Isopentylnitrit, die gasförmig oder Flüssigkeiten sind. Die Ester können als solche oder in einem Lösungsmittel gelöst, zum Beispiel in dem gleichen, in welchem die Reaktion abläuft, dem Reaktionsgemisch zugeführt werden; die niedrigsiedenden können auch in gasförmigem Zustand eingeleitet werden. Vorzugsweise wird Isopentyl- oder Methylnitrit verwendet. Die Diazotierung kann jedoch auch z.B. mit Nitrosylchlorid oder Nitrosylschwefelsäure durchgeführt werden. In einem

solchen Falle wird dann zweckmässig das so erhaltene Diazoniumsalz als Zwischenstufe isoliert,und die weitere Reaktionsfolge, ausgehend vom Diazoniumsalz, als Eintopfreaktion gemäss vorliegender Erfindung durchgeführt.

Im zweiten Reaktionsschritt (Meerwein-Reaktion) werden vorzugsweise solche Propenderivate der Formel $CH_2=C(CH_3)-Z$ eingesetzt, in denen Z ein CN-Rest oder die Acetatgruppe bedeutet, also Methacrylnitril oder Isopropenylacetat.

Als Ausgangssubstanzen zur Herstellung der Methoxybenzanthrone gemäss vorliegender Erfindung seien genannt: 4-Hydroxy-1-aminoanthrachinon; 5-Hydroxy-1-aminoanthrachinon; 5-Methoxy-1-aminoanthrachinon; 5-Chlor-1-aminoanthrachinon; 6,7-Dichlor-1-aminoanthrachinon sowie 5-Benzamido-1-aminoanthrachinon, insbesondere aber das unsubstituierte 1-Aminoanthrachinon.

Die Diazotierungsreaktion erfolgt bei einer etwas höheren Temperatur als bei gewöhnlichen Diazotierungen, nämlich im Bereich von etwa 30-80°C.

Die Umsetzung des 1-Anthrachinon-1-diazoniumsalzes mit dem Propenderivat (Meerwein-Reaktion) wird bei einer Temperatur von 20 bis 100°C durchgeführt, vorzugsweise simultan mit der Diazotierung, so dass das Diazoniumsalz stets nur in geringen Mengen vorhanden ist.

Die nachfolgende Stufe der Cyclisierung durch Einwirkung einer Base, vorzugsweise ein Alkalicarbonat, wird bei Temperaturen von 30-150°C, vorzugsweise bei 70-140°C, durchgeführt.

Die letzte Stufe der Methylierung erfolgt schliesslich bei 100-150°C, teilweise simultan mit der Cyclisierung.

Die Mengenverhältnisse der eingesetzten Reagenzien entsprechen zumindest den stöchiometrisch erforderlichen Mengen. Im Fall des Propenderivats ist es vorteilhaft, einen Ueberschuss von 1-5 Mol pro Mol 1-Aminoanthrachinon einzusetzen.

Das Kupfer-(I)-chlorid wird in katalytischen Mengen in der Grössenordnung von nur 0,003 bis 0,1 Mol, bezogen auf 1 Mol Anthrachinon-1-diazoniumsalz, angewandt.

Das als Reaktionsmedium und Methylierungsmittel verwandte Dimethylalkanphosphonat wird, bezogen auf die Ausgangsverbindung, in einem Gewichtsverhältnis von 2:1 bis 10:1 eingesetzt.

In einer zweckmässigen Ausführungsform wird das erfindungsgemässe Verfahren auf folgende Weise durchgeführt:

Die Hälfte des eingesetzten 1-Aminoanthrachinons wird in der dreifachen Gewichtsmenge Dimethylmethanphosphonat vorgelegt und mit Schwefelsäure 96%-ig versetzt. Nach Zugabe von Methacrylnitril wird auf 60-70°C erwärmt und Cu(I)-Chlorid, sowie etwa die Hälfte des Isopentylnitrits während 10-60 Minuten zugetropft. Es tritt sofort Stickstoffentwicklung ein.

Hierauf wird die zweite Hälfte des 1-Aminoanthrachinons und die 1,5-fache Gewichtsmenge Dimethylmethanphosphonat, sowie nochmals Cu(I)-Chlorid zugesetzt. Nun wird die zweite Hälfte des Isopentylnitrits zugetropft und ca. 10-30 Minuten nachgerührt. Durch diese Prozedur laufen Stufen 1 und 2 des angegebenen Formelschemas praktisch gleichzeitig ab.

Nachdem die Stickstoffentwicklung beendet ist, wird ein Alkalicarbonat, vorzugsweise Kaliumcarbonat, zugesetzt, auf ca. 140°C aufgeheizt und unter Rühren 1/4-1 Stunde bei 140-150°C gehalten, wobei die Stufen 3 und 4 teilweise gleichzeitig ablaufen.

Während des Aufheizens destilliert überschüssiges Methacrylnitril und der gebildete Isopentylalkol ab und es entwickelt sich $CO_2$. Nach beendeter Reaktion wird auf 100-130°C abgekühlt und durch Animpfen mit reinem 2-Methoxybenzanthron und weiteres Abkühlen bis auf 15°C das entstandene Produkt auskristallisiert.

Das Produkt wird abfiltriert, zuerst mit DMMP, dann mit Wasser gewaschen und gegebenenfalls getrocknet.

Das erfindungsgemässe Verfahren zur Herstellung von Methoxybenzanthronen stellt dank der Anwendung von Dimethylalkanphosphonaten und insbesondere von Dimethylmethanphosphonat als Reaktionsmedium, das gleichzeitig Methylierungsmittel ist, gegenüber dem Stand der Technik eine Vereinfachung der Reaktionsführung dar. Nach dem neuen Verfahren sind 2-Methoxybenzanthrone ohne Isolierung von Zwischenprodukten in einer Eintopfreaktion zugänglich, wobei das als Zwischenprodukt auftretende Diazoniumsalz stets nur in geringen Mengen vorliegt.

Gegenüber bekannten Verfahren werden höhere Produkt- und Raumzeitausbeuten erreicht. Da das Produkt zudem im angewandten Reaktionsmedium nur sehr gering löslich ist (1% bei Raumtemperatur) gestaltet sich die Produktisolierung sehr einfach.

Folgende Beispiele veranschaulichen das neue Verfahren. Die Temperaturen sind in Grad Celsius angegeben.

Beispiel 1: 33,8 g 1-Aminoanthrachinon 99%-ig werden in 100 ml Dimethylmethanphosphonat vorgelegt und unter Rühren innert 5 Min. mit 15,3 g Schwefelsäure 96%-ig versetzt. Nach Zugabe von 101 ml Methacrylnitril 99%-ig und Erwärmen auf 65° werden 0,1 g Cu (I)-Chlorid zugegeben und dann innert 15 Min. 18,5 g Isopentylnitrit 90%-ig bei 68-70° zugetropft, wodurch sofort Stickstoffentwicklung einsetzt. Nun werden weitere 33,8 g 1-Aminoanthrachinon 99%-ig, 50 ml Dimethylmethanphosphonat sowie 0,1 g Cu (I)-Chlorid zugegeben. Bei 68-72° werden 22,2 g Isopentylnitrit innert 15 Min. zugetropft, anschliessend wird 15 Min. bei 70-73° nachgerührt. Nach beendeter Gasentwicklung werden 66,3 g Kaliumcarbonat wasserfrei fein gemahlen innert 10 Min. eingetragen, der Ansatz innert 50 Min. auf 140° erwärmt und 1 Std. bei 140-145° gehalten. Dabei wird $CO_2$ frei und es destillieren insgesamt ca. 105 ml eines Gemisches von hauptsächlich Methacrylnitril und Isopentylalkohol ab. Man lässt das Reaktionsgemisch unter Rühren abkühlen und impft bei 120° mit 0,5 g 2-Methoxybenzanthron an. Ab 60° wird mittels Eiswasser auf 15° abgekühlt und 30 Min. bei 15° nachgerührt. Der Ansatz wird über eine Glasfritte unter Stickstoff abgedrückt, der Rückstand mit 100 ml Dimethylmethanphosphonat gewaschen und anschliessend mit Wasser neutral gewaschen. Nach dem Trocknen bei 80° unter Vakuum und Abzug von 0,5 g Impfkristallen erhält man 58,5 g 2-Methoxybenzanthron mit einem Gehalt von 98% entsprechend 56,7 g 2-Methoxybenzanthron 100%-ig = 73,5% der Theorie ber. auf eingesetztes 1-Aminoanthrachinon. Schmelzpunkt: 168-169°C. Sintert: >160°C.

Beispiel 2: In einem 200 ml Kolben werden zu einer Vorlage von 108 g Nitrosylschwefelsäure 38,8%-ig und 36,8 g Schwefelsäure 98%-ig 67,6 g 1-Aminoanthrachinon innerhalb von 1 Std. eingetragen. Die Temperatur lässt man nicht über 50° steigen. Die Lösung wird 30 Min. bei 45-50° nachgerührt, dann unter kräftigem Rühren auf 200 g Eis ausgetragen. Der beigefarbene Brei wird abgenutscht, gut abgepresst und mit 100 ml Eiswasser gewaschen (Feuchtgewicht ca. 150-160 g).

Das feuchte Anthrachinon-1-diazoniumhydrosulfat wird in 150 ml Dimethylmethanphosphonat in einem 500 ml Kolben eingetragen und mit 80,8 g Methacrylnitril versetzt. Unter Rühren werden bei 50-53° innerhalb von 1 Std. insgesamt 1,3 g Kupfer (I)-chlorid in Portionen zugegeben, wodurch Stickstoff und Blausäuregas entweichen. Nach 15-minütigem Nachrühren bei 50-55° wird die Apparatur auf 60 mm Hg evakuiert und überschüssiges Methacrylnitril bei 50-55° abdestilliert. Nach dem Belüften werden 124,4 g Kaliumcarbonat gemahlen innerhalb von 15 Min. eingetragen, wodurch $CO_2$-Gasentwicklung einsetzt. Ist die Zugabe beendet liegt die Temperatur bei 75°, es bildet sich ein dicker Brei. Das Oelbad wird innert 50 Min. auf 160° aufgeheizt. Hat die Reaktionsmasse 140° erreicht, lässt man 30 Min. bei 140-5° nachrühren. Man lässt abkühlen und tropft bei 100° langsam 150 ml Wasser zu. Der abgekühlte Ansatz wird abfiltriert und mit ca. 600 ml Wasser neutral gewaschen. Nach Trocknung bei 80° im Vakuum erhält man 77-80 g rohes 2-Methoxybenzanthron mit einem Gehalt von 77-80% entsprechend einer Ausbeute an reinem 2-Methoxybenzanthron von 77-80% d. Th. (ber. auf eingesetztes 1-Aminoanthrachinon).

Reinigung: Das getrocknete Rohprodukt wird anschliessend in 50 ml Dimethylmethanphosphonat angeschlämmt und 30 Min. gerührt. Die Anschlämmung wird abfiltriert, mit 50 ml Dimethylmethanphosphonat und danach ca. 150 ml Wasser gewaschen und bei 80° im Vakuum getrocknet. Man erhält 56-59 g 2-Methoxybenzanthron entsprechend 71,5-75,5% der Theorie (berechnet auf eingesetztes 1-Aminoanthrachinon) mit einem Gehalt von 96-99%.

Beispiel 3: 11,4 g 1-Aminoanthrachinon 98%-ig werden in 20 ml Dimethylmethanphosphonat (DMMP) suspendiert und mit 8,4 ml Isopentylnitrit 90%-ig versetzt. Bei 20-30° werden innert 1 Std. 2,92 ml Schwefelsäure 96%-ig zugetropft. Nun wird auf 55° aufgeheizt und 1/2 Std. bei dieser Temperatur gerührt. Nach Zugabe von 21 ml Methacrylnitril

werden innert 20 Min. bei 55-60°C portionenweise insgesamt 150 mg Cu (I)-chlorid eingetragen, wodurch Stickstoffentwicklung eintritt. Anschliessend wird bei 40 mm Hg-Vakuum überschüssiges Methacrylnitril abdestilliert. Es werden daraufhin 30 ml Dimethylmethanphosphonat und 20,7 g Kaliumcarbonat zugegeben. Das Reaktionsgemisch wird innert 15 Min. auf 140° aufgeheizt und 1 Std. bei 140-145° nachgerührt, dann lässt man abkühlen und gibt bei 100° 100 ml Wasser zu. Der Ansatz wird bei Raumtemperatur abgenutscht und mit Wasser neutral gewaschen. Das getrocknete rohe 2-Methoxybenzanthron (13,2 g) wird in 20 ml DMMP angeschlämmt, 30 Min. gerührt, abgenutscht, mit 20 ml DMMP und anschliessend mit Wasser gewaschen. Nach Trocknung bei 60° unter Vakuum erhält man 10,1 g 2-Methoxybenzanthron mit einem Gehalt von 86,5%, entsprechend 8,74 g 2-Methoxybenzanthron 100%-ig = 67,3% d. Th. berechnet auf eingesetztes 1-Aminoanthrachinon.

Beispiel 4: In eine Vorlage von 50 ml Dimethylmethanphosphonat werden unter Niveau 4,2 g Nitrosylchlorid eingeleitet. In die gelbbraune Lösung werden bei 20°C innert 1 Std. 11,4 g 1-Aminoanthrachinon eingetragen. Die beigefarbene Suspension wird 1 Std. bei 20-25°C und dann 3/4 Std. bei 50° gerührt. Nun werden 21 ml Methacrylnitril zugegeben und bei 50-55°C innert 45 Min. 8 Portionen zu je 50 mg = 0,4 g $Cu_2Cl_2$ eingetragen, wodurch Stickstoffentwicklung einsetzt. Nach beendeter Gasentwicklung wird auf 60 mm Hg-Vakuum evakuiert und überschüssiges Methacrylnitril bei 50-55°C abdestilliert. Nach dem Belüften werden bei 65°C 15 ml Dimethylmethanphosphonat und 17,3 g Kaliumcarbonat zugegeben. Man erwärmt innert 30 Min. auf 140°, wobei $CO_2$- und HCN-Gas entweichen. Nach 20-minütigem Nachrühren bei 140-145°C lässt man abkühlen, und tropft bei 100°C langsam 75 ml Wasser zu. Der abgekühlte Ansatz wird filtriert und mit ca. 100 ml Wasser neutral gewaschen. Nach Trocknung bei 80°C unter Vakuum erhält man 14,3 g rohes 2-Methoxybenzanthron.

<u>Beispiel 5:</u> In eine Vorlage von 36 g Nitrosylschwefelsäure 38,8%-ig und 12,3 g Schwefelsäure 98%-ig werden 22,5 g 1-Aminoanthrachinon innerhalb von 1 Std. eingetragen. Die Temperatur lässt man nicht über 50°C steigen. Die Lösung wird 30 Min. bei 45-50°C nachgerührt, dann unter kräftigem Rühren auf 70 g Eis ausgetragen. Der beigefarbene Brei wird abgenutscht, gut abgepresst und mit 35 ml Eiswasser gewaschen.
Das feuchte Anthrachinon-1-diazoniumhydrosulfat wird in 50 ml Dimethylmethanphosphonat angeschlämmt. Nach Zugabe von 22,2 ml Isopropenylacetat werden innert 1 1/2 Std. bei 25-35°C in Abständen von 9 Min. insgesamt 11 Portionen à 0,1 g Cu (I)-chlorid zugegeben, wodurch Stickstoff freigesetzt wird. Anschliessend wird auf 50-55°C erwärmt und unter langsamem Evakuieren bis zu einem Endvakuum von 13 mm Hg und einer Temperatur von 70°C total ca. 20 ml Destillat erhalten. Nach dem Belüften werden 55,2 g Kaliumcarbonat eingetragen. Der Ansatz wird innert 1 Std. auf 140°C aufgeheizt und 45 Min. bei 140-145°C gehalten, wobei $CO_2$ entweicht und insgesamt weitere 10 ml Destillat anfallen. Man lässt abkühlen und tropft bei 100°C 50 ml Wasser zu. Nach Abkühlen auf Raumtemperatur wird das Rohprodukt abfiltriert, mit Wasser neutral gewaschen und bei 60°C unter Vakuum getrocknet. Das trockene Rohprodukt (~25,4 g) wird in 15 ml Dimethylmethanphosphonat eingetragen und 30 Min. bei Raumtemperatur gerührt. Durch Filtration, Waschen des Rückstandes mit 15 ml Dimethylmethanphosphonat und anschliessend Wasser, erhält man 21,3 g 2-Methoxybenzanthron mit einem Gehalt von 90%, entsprechend 19,1 g reinem 2-Methoxybenzanthron = 73,6% d. Th. berechnet auf eingesetztes 1-Aminoanthrachinon.

<u>Beispiel 6:</u> In einem 500 ml Kolben wird ein Gemisch aus 100 ml Dimethylmethanphosphonat, 83,3 g Methacrylnitril 99%ig und 33,8 g 1-Aminoanthrachinon 99%ig vorgelegt. Unter Rühren werden 24,6 g 60%ige Schwefelsäure zugetropft. Die dunkelrote Suspension wird auf 65° aufgeheizt. Nach Zugabe von 0,1 g Kupfer(I)chlorid wird bei 65-68° innerhalb von 40 Minuten Methylnitrit (hergestellt durch Zutropfen von 19,3 g 40%iger Schwefelsäure zu einer Lösung von 24 g Natriumnitrit in 12 g Methanol und 10 ml Wasser) unter Niveau eingeleitet. Stick-

stoffentwicklung zeigt das augenblickliche Einsetzen der Reaktion an. Nach 10 minütigem Nachrühren werden weitere 33,7 g 1-Aminoanthrachinon zusammen mit 0,1 g Kupfer(I)chlorid zum Reaktionsgemisch gegeben. Bei 68-71° wird innerhalb von 50 Minuten erneut Methylnitrit eingeleitet.

Nach beendeter Stickstoffentwicklung destilliert man aus dem Reaktionsgemisch bei einer Temperatur von 80° und einem Druck von 80 mbar ca. 78 g eines Gemisches von Methanol und Methacrylnitril ab. Anschliessend werden bei 75° 50 ml Dimethylmethanphosphonat zugegeben und dann 27,9 g Kaliumcarbonat eingetragen, wobei Kohlendioxid entweicht.

Man erhitzt das Reaktionsgemisch anschliessend auf 100° und trägt innerhalb von 15 Minuten weitere 39 g Kaliumcarbonat ein, wodurch die Innentemperatur auf 130° steigt. Dann wird schliesslich bei 140° ca. 30 Minuten nachgerührt, danach lässt man auf 15° abkühlen. Bei einer Temperatur von 120° werden 0,5 g Methoxybenzanthron (Impfkristalle) zugegeben.

Man rührt das Reaktionsgemisch 1 Stunde bei 15°, drückt dann den dunkelbraunen Kristallbrei über eine Glasfritte ab und wäscht mit 100 ml Dimethylmethanphosphonat nach. Mit weiteren 500 ml Wasser wird salzfrei und neutral gewaschen, dann trocken gesaugt und das Produkt bei 80°C im Vakuum getrocknet. Man erhält 55-59 g 2-Methoxybenzanthron mit einer Reinheit von 96-99 %; Ausbeute 70-73 %; Mp. 168-9°C.

Patentansprüche

1. Verfahren zur Herstellung von 2-Methoxybenzanthronen ausgehend von 1-Aminoanthrachinon der Formel I

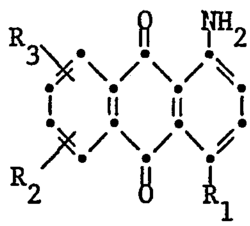

(I)

worin $R_1$, $R_2$ und $R_3$ Wasserstoff, Chlor, Hydroxy, Methoxy oder die Benzamidogruppe bedeuten, das umgesetzt wird zum Anthrachinon-1-diazoniumsalz, welches man mit einem Propenderivat der Formel $CH_2=C(CH_3)-Z$ reagieren lässt, wobei Z ein elektronenziehender Substituent ist; das Reaktionsprodukt cyclisiert im alkalischen Reaktionsgemisch zum 2-Hydroxybenzanthron, dessen Hydroxygruppe unter Einwirkung des Reaktionsmediums zum 2-Methoxybenzanthron methyliert wird, dadurch gekennzeichnet, dass man die Reaktion ausgehend von 1-Aminoanthrachinon der Formel I oder vom entsprechenden Anthrachinon-1-diazoniumsalz, ohne Isolieren von Zwischenprodukten, in einem Dimethylalkanphosphonat durchführt, dessen Alkanrest 1-4 C-Atome aufweist.

2. Verfahren nach Anspruch 1, dadurch gekennzeichnet, dass man von 1-Aminoanthrachinonen der Formel I ausgeht.

3. Verfahren nach Anspruch 1, dadurch gekennzeichnet, dass man Dimethylmethanphosphonat als Reaktionsmedium verwendet.

4. Verfahren nach Anspruch 1, dadurch gekennzeichnet, dass man die Diazotierung und die Meerwein-Reaktion simultan durchführt.

5. Verfahren nach Anspruch 1, dadurch gekennzeichnet, dass man von unsubstituiertem 1-Aminoanthrachinon ausgeht.

6. Verfahren nach Anspruch 1, dadurch gekennzeichnet, dass man zur Diazotierung die Salpetrigsäureester von Alkoholen mit 1-5 Kohlenstoffatomen oder Nitrosylchlorid oder Nitrosylschwefelsäure verwendet.

7. Verfahren nach Anspruch 1, dadurch gekennzeichnet, dass man das Anthrachinon-1-diazoniumsalz mit einem Propenderivat der Formel $CH_2=C(CH_3)-Z$ umsetzt, in dem Z die Cyanogruppe oder einen Acetatrest bedeutet.

8. Verfahren nach Anspruch 7, dadurch gekennzeichnet, dass man Methacrylnitril verwendet.

9. Verfahren nach Anspruch 1, dadurch gekennzeichnet, dass man die Cyclisierungsreaktion mittels Alkalicarbonaten durchführt.

10. Verfahren nach Anspruch 9, dadurch gekennzeichnet, dass man wasserfreies Kaliumcarbonat verwendet.

11. Verfahren nach Anspruch 1, dadurch gekennzeichnet, dass man die Diazotierung bei einer Temperatur zwischen 30 und 80°C, die Meerweinreaktion bei einer Temperatur zwischen 20 und 100°C und die Cyclisierung sowie Methylierung bei einer Temperatur zwischen 30 und 150°C durchführt.

12. Verfahren nach Anspruch 1, dadurch gekennzeichnet, dass man das Dimethylalkanphosphonat bezogen auf das 1-Aminoanthrachinon in einem Gewichtsverhältnis von 2:1 bis 10:1 verwendet.

13. Die gemäss dem Verfahren nach Anspruch 1 erhaltenen 2-Methoxybenzanthrone.

0050872

Europäisches Patentamt

# EUROPÄISCHER RECHERCHENBERICHT

Nummer der Anmeldung
EP 81 10 8925

| EINSCHLÄGIGE DOKUMENTE | | | KLASSIFIKATION DER ANMELDUNG (Int. Cl.3) |
|---|---|---|---|
| Kategorie | Kennzeichnung des Dokuments mit Angabe, soweit erforderlich, der maßgeblichen Teile | betrifft Anspruch | |
| Y | FR - A - 2 315 496 (MONTEDISON)<br>* Ansprüche 1-10; Seite 2, Zeile 15 - Seite 5, Zeile 6 *<br>& DE - A - 2 627 867<br>-- | 1,2,5,<br>7-10 | C 07 C 49/83<br>C 09 B 3/04 |
| Y | FR - A - 2 414 489 (CIBA-GEIGY)<br>* Ansprüche 1-5 *<br>& DE - A - 2 900 630<br>---- | 1,3 | |

RECHERCHIERTE SACHGEBIETE (Int. Cl.3)

C 09 B 3/04
C 07 C 49/83
C 09 B 3/02
3/00
C 07 C 49/788
49/782

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

✓ Der vorliegende Recherchenbericht wurde für alle Patentansprüche erstellt.

| Recherchenort | Abschlußdatum der Recherche | Prüfer |
|---|---|---|
| Den Haag | 01-02-1982 | DELANGHE |

EPA form 1503.1 06.78